# FASCICULE DE BREVET EUROPEEN

(11) **EP 0 648 107 B1**
(45) Date de publication et mention de la délivrance du brevet: **07.10.1998**
(21) Numéro de dépôt: 94915591.5
(22) Date de dépôt: 06.05.1994
(51) Int. Cl.: A61K 7/06

(54) **COMPOSITION POUR DIMINUER LA CHUTE DES CHEVEUX ET/OU STIMULER LEUR REPOUSSE**
ZUSAMMENSETZUNG FÜR DIE VERHÜTUNG VON HAARAUSFALL UND/ODER FÜR DIE WACHSTUMSFÖRDERUNG VON HAAREN
COMPOSITION FOR REDUCING HAIR LOSS OR STIMULATING HAIR GROWTH

(30) Priorité: 07.05.1993 FR 9305522
(43) Date de publication de la demande: 19.04.1995
(73) Titulaire: L'OREAL, 75008 Paris (FR)
(72) Inventeur: PARADOT, France, F-92500 Rueil-Malmaison (FR)
(74) Mandataire: Casalonga, Axel
(86) Numéro de dépôt international: FR9400527
(87) Numéro de publication internationale: WO9426239

(56) Documents cités:
- EP-A- 0 383 467
- WO-A-92/17153
- WO-A-92/21318
- FR-A- 2 609 393
- US-A- 2 383 990

## Description

L'invention est relative à des compositions destinées à favoriser la repousse des cheveux et/ou diminuer la chute des cheveux, à base de fructose, glucose et de protéines globulaires de céréale ou leurs hydrolysats.

L'homme de l'art sait depuis longtemps que la chute naturelle des cheveux chez l'homme reflète globalement l'équilibre des follicules pileux entre les phases alternatives de croissance (anagène) et les phases de chute (télogène).

Le rapport moyen du nombre de follicules en phase anagène sur celui en phase télogène est de l'ordre de 9 (90/10). Le pourcentage de follicules en phase de repos (catagène) y apparaît comme étant très faible.

La chute ou perte naturelle des cheveux peut être estimée, à quelques cent cheveux par jour peur l'état physiologique normal.

Il est connu, par ailleurs, que certains facteurs tels qu'un déséquilibre hormonal, un stress physiologique, la malnutrition, peuvent accentuer le phénomène.

Dans certaines dermatoses du cuir chevelu à caractéristique inflammatoire, telles que par exemple le psoriasis ou les dermatites séborrhéiques, la chute des cheveux peut être fortement accentuée ou entraîner des cycles des follicules fortement perturbés.

De façon surprenante, la demanderesse a découvert des compositions à base de fructose, de glucose et de protéines globulaires de céréale ou leurs hydrolysats, permettant de prolonger la survie du follicuie pileux pendant la phase anagène.

La demanderesse a constaté que ces compositions étaient particulièrement efficaces pour la repousse des cheveux et/ou pour freiner la chute des cheveux, notamment dans les traitements des maladies du cuir chevelu, telles que la pelade, la chute des cheveux, la dermatite desquamante, l'alopécie.

Selon une forme de réalisation particulièrement préférée, on a constaté que l'utilisation d'un agent hydratant dans ces compositions permettait d'améliorer leur efficacité.

L'invention a donc peur objet une nouvelle composition pour diminuer la chute des cheveux et/ou pour favoriser la repousse des cheveux.

Un autre objet de l'invention est constitué par son application au traitement du cuir chevelu et des cheveux.

Un objet de l'invention est également constitué par l'utilisation des compositions telles que définies ci-dessus pour la préparation d'un médicament destiné au traitement des maladies du cuir chevelu, telles que la chute des cheveux, la dermatite desquamante, l'alopécie, la pelade.

L'invention a enfin pour objet un procédé non-thérapeutique de traitement des cheveux, consistant à appliquer au moins une des compositions telles que définies ci-dessus sur le cuir chevelu ou les cheveux, puis éventuellement à les rincer à l'eau; la composition ayant essentiellement dans ce but un effet d'amélioration de l'aspect de la chevelure.

D'autres objets de l'invention apparaîtront à la lecture de la description et des exemples qui suivent.

La composition conforme à l'invention est essentiellement caractérisée par le fait qu'elle contient dans un milieu aqueux ou hydroalcoolique comprenant de l'eau et de 0,01 à 0,5% en poids d'alkylèneglycol en C₃-C₆ ou de polyalkylèneglycol en C₂-C₄ liquide à température ambiante :
a) au moins de 0,03 à 0,3% en poids de fructose;
b) au moins de 0,002 à 0,05% en poids de glucose;
c) au moins de 0,03 à 0,2% en poids d'une ou plusieurs protéines globulaires de céréale ou leurs hydrolysats;
d) au moins de 0,03 à 1% en poids d'un sel de sodium ou de potassium;
les pourcentages en poids étant définis par rapport au poids total de la composition.

Une forme particulièrement préférée de composition selon l'invention consiste en une formulation telle que définie précédemment, contenant en plus un agent hydratant choisi parmi l'urée et un polyol en C₃-C₆ tel que le sorbitol, la glycérine. L'agent hydratant est utilisé dans des proportions comprises de préférence entre 0,005 et 0,5% en poids par rapport au poids total de la composition. On utilise plus particulièrement l'urée.

Les compositions préférentielles conformes à l'invention contiennent un acide organique tel que l'acide lactique ou l'acide citrique. Le pH final de la composition a une valeur comprise entre 6,5 et 8 et est ajusté par tout agent acidifiant et/ou alcalinisant. L'acide lactique est plus particulièrement utilisé par exemple dans des concentrations comprises entre 2.10⁻⁴ et 1% en poids.

Les protéines globulaires de céréale utilisées conformément à la présente invention, sont choisies notamment parmi les sphéroprotéines de blé, de maïs, d'orge, d'avoine, de riz, de millet. Ces protéines de céréales sont constituées notamment d'un mélange de prolamine et de gluténine.

La prolamine de blé est la gliadine; celle de l'orge est l'hordeïne; celle de l'avoine l'avenine; celle du mais la zeïne; celle du riz l'orizine; celle du millet la kafirine.

Ces sphéroprotéines sont principalement constituées d'acide glutamique, de proline et d'une faible quantité de lysine.

Le poids moléculaire des sphéroprotéines de céréales utilisées selon l'invention, est de préférence supérieur à 20.000.

Le poids moléculaire des hydrolysats de ces protéines est de préférence inférieur à 20.000.

Les protéines ou les hydrolysats des protéines de l'invention sont utilisés préférentiellement dans des quantités comprises entre 0,05 et 0,15% en poids.

On utilise plus particulièrement des sphéroprotéines de blé de poids moléculaire supérieur à 30.000 et dont le taux de pureté protéique est supérieur à 90%.

Les solvants utilisés dans le milieu aqueux ou hydroalcoolique des compositions de l'invention, sont choisis notamment parmi les polyéthylèneglycols, notamment les polyéthylèneglycols 300, 400 et 600; le propylèneglycol, l'hexylèneglycol et le dipropylèneglycol.

Les alcools utilisés dans les compositions hydroalcooliques sont des alcools en C₂-C₃, notamment l'éthanol et le propanol. Ils sont présents dans des quantités de 25 à 50% en poids par rapport au poids du mélange eau/alcool.

Les sels présents dans les compositions de l'invention sont choisis notamment parmi le chlorure de sodium et le chlorure de potassium et plus particulièrement le chlorure de sodium. Ils sont utilisés préférentiellement dans des quantités comprises entre 0,1 et 0,5% en poids.

Le fructose et le glucose sont utilisés de manière préférentielle dans des proportions comprises respectivement entre 0,05 et 0,2% en poids et entre 0,01 et 0,03% en poids.

Les compositions selon l'invention peuvent se présenter sous des formes galéniques diverses habituellement utilisées en pharmacie ou en cosmétique pour le traitement du cuir chevelu.

Elles se présentent en particulier sous forme de lotion épaissie ou non que l'on utilise en traitement rincé ou non rincé; de gel; de crème ou de lait rincé; de mousse, de spray à jet directionnel.

Ces compositions peuvent contenir des agents épaississants tels que la cellulose ou des dérivés de cellulose comme l'hydroxyéthylcellulose; des hétérobiopolysaccharides comme la gomme de xanthane; la gomme de guar; des acides polyacryliques réticulés par un agent polyfonctionnel tel que les produits vendus sous la dénomination CARBOPOL, des polymères ou copolymères réticulés d'acrylamide.

Ces compositions peuvent éventuellement renfermer d'autres adjuvants habituellement utilisés en cosmétique ou en pharmacie, notamment au niveau du cuir chevelu, tels que des agents tensio-actifs, des parfums, des agents conservateurs, des colorants, des polymères cationiques, non-ioniques, anioniques ou amphotères, des agents propulseurs, des stabilisateurs de mousse, des oligo-éléments, des agents antipelliculaires, des apaisants, des agents antibactériens, des agents stimulant le cuir chevelu tels que les vitamines comme le penthoténate de calcium, des agents antigras.

Les exemples qui suivent servent à illustrer l'invention sans toutefois présenter un caractère limitatif.

### EXEMPLE 1

| **LOTION ANTICHUTE** | |
|---|---|
| - Protéine de blé | 0,12 g |
| - Fructose | 0,22 g |
| - Glucose | 0,04 g |
| - Urée | 0,06 g |
| - NaCl | 0,5 g |
| - Polyéthylèneglycol | 0,25 g |
| - Acide lactique | 0,05 g |
| - Potasse qs pH = 7,5 | |
| - Eau/alcool éthylique à 31% en poids | qsp 100 g |

### EXEMPLE 2

| **LOTION EPAISSIE ANTICHUTE** | |
|---|---|
| - Protéine de blé | 0,09 g |
| - Fructose | 0,15 g |
| - Glucose | 0,02 g |
| - Urée | 0,03 g |
| - NaCl | 0,3 g |
| - Propylèneglycol | 0,15 g |
| - Acide lactique | 0,01 g |
| - Potasse qs pH = 7,5 | |
| - Gomme de guar vendue sous la dénomination de "JAGUAR HP8" par la Société MEYHALL | 0,4 g |
| - Eau/alcool éthylique à 31% en poids | qsp 100 g |

## Revendications

1. Composition destinée à favoriser la repousse des cheveux et/ou à freiner la chute des cheveux, caractérisée par le fait qu'elle contient dans un milieu aqueux ou hydroalcoolique comprenant de l'eau et de 0,01 à 0,5% en poids d'un alkylèneglycol en C₃-C₆ ou d'un polyaylèneglycol en C₂-C₄ liquide à température ambiante :
a) au moins de 0,03 à 0,3% en poids de fructose;
b) au moins de 0,002 à 0,05% en poids de glucose;
c) au moins de 0,03 à 0,2% en poids d'une ou plusieurs sphéroprotéines de céréale ou leurs hydrolysats;
d) au moins de 0,03 à 1% en poids d'un sel de potassium ou de sodium;
les pourcentages en poids étant définis par rapport au poids total de la composition.

2. Composition selon la revendication 1, caractérisée par le fait qu'elle contient en plus un agent hydratant choisi parmi l'urée ou un polyol en C₃-C₆.

3. Composition selon la revendication 1 ou 2, caractérisée par le fait qu'elle contient en plus un acide organique.

4. Composition selon la revendication 2, caractérisée par le fait que l'agent hydratant est présent dans des proportions comprises entre 0,005 et 0,5% en poids.

5. Composition selon la revendication 2 ou 4, caractérisée par le fait que l'agent hydratant est l'urée.

6. Composition selon la revendication 3, caractérisée par le fait que l'acide organique est l'acide lactique ou citrique.

7. Composition selon l'une quelconque des revendications 1 à 6, caractérisée par le fait que les sphéroprotéines de céréale ou leurs hydrolysats sont choisis parmi les sphéroprotéines de blé, mais, avoine, orge, riz ou millet, ou leurs hydrolysats.

8. Composition selon la revendication 7, caractérisée par le fait que les sphéroprotéines ont un poids moléculaire supérieur à 20.000 et leurs hydrolysats ont un poids moléculaire inférieur à 20.000.

9. Composition selon l'une quelconque des revendications 1 à 8, caractérisée par le fait qu'elle contient des sphéroprotéines de blé de poids moléculaire supérieur à 30.000.

10. Composition selon l'une quelconque des revendications 1 à 9, caractérisée par le fait que le milieu aqueux ou hydroalcoolique contient un solvant choisi parmi le polyéthylèneglycol, le propylèneglycol, l'hexylèneglycol et le dipropylèneglycol.

11. Composition selon l'une quelconque des revendications 1 à 10, caractérisée par le fait que le milieu hydroalcoolique contient de 25 à 50% en poids d'alcool en C₂-C₅ par rapport au poids total dudit milieu.

12. Composition selon l'une quelconque des revendications 1 à 10, caractérisée par le fait que le sel de sodium ou de potassium est le chlorure de sodium ou de potassium.

13. Composition selon l'une quelconque des revendications 1 à 12, caractérisée par le fait qu'elle contient :
a) de 0,05 à 0,2% en poids de fructose;
b) de 0,01 à 0,03% en poids de glucose;
c) de 0,05 à 0,15% en poids de protéines de céréales ou d'hydrolysat de protéine globulaire;
d) de 0,1 à 0,5% en poids de sel de sodium ou de potassium.

14. Composition selon l'une quelconque des revendications 1 à 13, caractérisée par le fait qu'elle se présente sous forme de lotion épaissie ou non, de gel, de crème ou de lait rincé, de mousse ou de spray à jet directionnel.

15. Composition selon l'une quelconque des revendications 1 à 14, caractérisée par le fait qu'elle contient en plus des agents épaississants, des agents tensio-actifs, des conservateurs, des parfums, des colorants, des stabilisateurs de mousse, des agents propulseurs, des polymères catiouiques, anioniques, non-ioniques ou amphotères, des oligo-éléments, des agents antipelliculaires, des apaisants, des agents antibactériens, des agents stimulant le cuir chevelu, des agents antigras.

16. Procédé de traitement non-thérapeutique des cheveux, caractérisé par le fait que l'on applique une composition selon l'une quelconque des revendications 1 à 15, sur le cuir chevelu ou les cheveux; ladite application étant éventuellement suivie d'un rinçage à l'eau.

17. Utilisation d'une composition selon l'une quelconque des revendications 1 à 15, pour la préparation d'un médicament pour le traitement de la chute des cheveux, de la pelade, de la dermatite desquamante ou de l'alopécie.

## Claims

1. Composition intended for promoting hair regrowth and/or halting hair loss, characterized in that it contains, in an aqueous or aqueous-alcoholic medium comprising water and from 0.01 to 0.5 % by weight of a C₃-C₆ alkylene glycol or of a C₂-C₄ polyalkylene glycol which is liquid at room temperature:
a) at least from 0.03 to 0.3 % by weight of fructose;
b) at least from 0.002 to 0.05 % by weight of glucose;
c) at least from 0.03 to 0.2 % by weight of one or more cereal globular proteins or their hydrolysates;
d) at least from 0.03 to 1 % by weight of a sodium or potassium salt;
the weight percentages being defined relative to the total weight of the composition.

2. Composition according to Claim 1, characterized in that it contains, in addition, a hydrating agent chosen from urea and a C₃-C₆ polyol.

3. Composition according to Claim 1 or 2, characterized in that it contains, in addition, an organic acid.

4. Composition according to Claim 2, characterized in that the hydrating agent is present in proportions of betwen 0.005 and 0.5 % by weight.

5. Composition according to Claim 2 or 4, characterized in that the hydrating agent is urea.

6. Composition according to Claim 3, characterized in that the organic acid is lactic or citric acid.

7. Composition according to any one of Claims 1 to 6, characterized in that the cereal globular proteins or their hydrolysates are chosen from the globular proteins of wheat, maize, oats, barley, rice or millet, or their hydrolysates.

8. Composition according to Claim 7, characterized in that the globular proteins have a molecular weight of greater than 20,000, and their hydrolysates have a molecular weight of less than 20,000.

9. Composition according to any one of Claims 1 to 8, characterized in that it contains wheat globular proteins of molecular weight greater than 30,000.

10. Composition according to any one of Claims 1 to 9, characterized in that the aqueous or aqueous-alcoholic medium contains a solvent chosen from polyethylene glycol, propylene glycol, hexylene glycol and dipropylene glycol.

11. Composition according to any one of Claims 1 to 10, characterized in that the aqueous-alcoholic medium contains from 25 to 50 % by weight of C₂-C₅ alcohol relative to the total weight of the said medium.

12. Composition according to any one of Claims 1 to 10, characterized in that the sodium or potassium salt is sodium or potassium chloride.

13. Composition according to any one of Claims 1 to 12, characterized in that it contains:
a) from 0.05 to 0.2 % by weight of fructose;
b) from 0.01 to 0.03 % by weight of glucose;
c) from 0.05 to 0.15 % by weight of cereal proteins or of hydrolysate of globular protein;
d) from 0.1 to 0.5 % by weight of sodium salt or potassium salt.

14. Composition according to any one of Claims 1 to 13, characterized in that it takes the form of a lotion, thickened or otherwise, gel, rinsed milk or cream, mousse or spray with a directional nozzle.

15. Composition according to any one of Claims 1 to 14, characterized in that it contains, in addition, thickeners, surfactants, preservatives, perfumes, colorants, foam stabilizers, propellants, cationic, anionic, nonionic or amphoteric polymers, trace elements, anti-dandruff agents, soothing agents, antibacterial agents, scalp-stimulating agents or anti-grease agents.

16. Process for the non-therapeutic treatment of hair, characterized in that a composition according to any one of Claims 1 to 15 is applied to the scalp or hair, the said application being followed, where appropriate, by a rinse with water.

17. Use of a composition according to any one of Claims 1 to 15, for the preparation of a medicinal product for the treatment of hair loss, pelade, desquamating dermatitis or alopecia.

## Patentansprüche

1. Zusammensetzung zur Begünstigung des erneuten Wachstums der Haare und/oder zur Hemmung des Ausfalls der Haare, dadurch gekennzeichnet, daß sie in einem wässrigen oder hydroalkoholischen Milieu aus Wasser und aus 0,01 bis 0,5 Gew.% C₃₋₆-Alkylenglycol oder bei Umgebungstemperatur flüssigem C₂₋₄-Polyalkylenglycol enthält:
a) mindestens 0,03 bis 0,3 Gew.% Fructose,
b) mindestens 0,002 bis 0,05 Gew.% Glucose,
c) mindestens 0,03 bis 0,2 Gew.% eines oder mehrerer globulärer Proteine von Getreide oder von deren Hydrolysaten,
d) mindestens 0,03 bis 1 Gew.% eines Natrium- oder Kaliumsalzes,
wobei die Gewichtsprozentangaben auf das Gesamtgewicht der Zusammensetzung bezogen sind.

2. Zusammensetzung gemäß Anspruch 1, dadurch gekennzeichnet, daß sie ausserdem ein Hydratisiermittel enthält, ausgewählt aus Harnstoff oder einem C₃₋₆-Polyol.

3. Zusammensetzung gemäß Anspruch 1 oder 2, dadurch gekennzeichnet, daß sie ausserdem eine organische Säure enthält.

4. Zusammensetzung gemäß Anspruch 2, dadurch gekennzeichnet, daß das Hydratisiermittel in Mengenanteilen von 0,005 bis 0,5 Gew.% vorhanden ist.

5. Zusammensetzung gemäß Anspruch 2 oder 4, dadurch gekennzeichnet, daß das Hydratisiermittel Harnstoff ist.

6. Zusammensetzung gemäß AnspruCh 3, dadurch gekennzeichnet, daß die organische Säure Milch- oder Zitronensäure ist.

7. Zusammensetzung gemäß einem der Ansprüche 1 bis 6, dadurch gekennzeichnet, daß die Sphäroproteine von Getreide oder deren Hydrolysate aus den Sphäroproteinen von Weizen, Mais, Hafer, Gerste, Reis oder von Hirse oder aus deren Hydrolysaten ausgewählt sind.

8. Zusammensetzung gemäß Anspruch 7, dadurch gekennzeichnet, daß die Sphäroproteine ein Molekulargewicht von mehr als 20000 und deren Hydrolysate ein Molekulargewicht von weniger als 20000 aufweisen.

9. Zusammensetzung nach einem der Ansprüche 1 bis 8, dadurch gekennzeichnet, daß sie Sphäroproteine von Weizen eines Molekulargewichts von mehr als 30000 enthält.

10. Zusammensetzung gemäß einem der Ansprüche 1 bis 9, dadurch gekennzeichnet, daß das wässrige oder hydroalkoholische Milieu ein Lösungsmittel enthält, das aus Polyethylenglycol, Propylenglycol, Hexylenglycol und aus Dipropylenglycol ausgewählt ist.

11. Zusammensetzung nach einem der Ansprüche 1 bis 10, dadurch gekennzeichnet, daß das hydroalkoholische Milieu 25 bis 50 Gew.% C₂₋₅-Alkohol, bezogen auf das Gesamtgewicht des gesamten Milieu, enthält.

12. Zusammensetzung gemäß einem der Ansprüche 1 bis 10, dadurch gekennzeichnet, daß das Natrium- oder Kaliumsalz Natrium- oder Kaliumchlorid ist.

13. Zusammensetzung gemäß einem der Ansprüche 1 bis 12, dadurch gekennzeichnet, daß sie enthält:
a) 0,05 bis 0,2 Gew.% Fructose,
b) 0,01 bis 0,03 Gew.% Glucose,
c) 0,05 bis 0,15 Gew.% Proteine von Getreidesorten oder Hydrolysat von globulärem Protein,
d) 0,1 bis 0,5 Gew.% Natrium- oder Kaliumsalz.

14. Zusammensetzung gemäß einem der Ansprüche 1 bis 13, dadurch gekennzeichnet, daß sie in Form einer gegebenenfalls verdickten Lotion, eines Gel, einer Creme oder Milch zur Spülung, eines Schaums oder eines Spray mit Richtungsstrahl vorliegt.

15. Zusammensetzung gemäß einem der Ansprüche 1 bis 14, dadurch gekennzeichnet, daß sie ausserdem Verdickungsmittel, oberflächenaktive Mittel, Konservierungsmittel, ParfümProdukte, Färbemittel, Schaumstabilisatoren, Treibmittel, kationische, anionische, nicht-ionische oder amphotere Polymere, Oligoelemente, Antischuppenmittel, Beruhigungsmittel, antibakterielle Mittel, die behaarte Haut stimulierende Mittel und Antifettmittel enthält.

16. Verfahren zur nicht-therapeutischen Behandlung der Haare, dadurch gekennzeichnet, daß man eine Zusammensetzung gemäß einem der Ansprüche 1 bis 15 auf die behaarte Haut oder die Haare anwendet und/oder aufbringt, worauf auf die genannte Anwendung gegebenenfalls eine Spülung mit Wasser erfolgt.

17. Verwendung einer Zusammensetzung gemäß einem der Ansprüche 1 bis 15 zur Herstellung eines Medikaments zur Behandlung des Ausfalls der Haare, von Haarschwund, Schuppen bildender Dermatitis oder von Alopezie.
